# FASCICULE DE BREVET EUROPEEN

(11) **EP 0 671 397 B1**
(45) Date de publication et mention de la délivrance du brevet: **03.02.1999**
(21) Numéro de dépôt: 95400496.6
(22) Date de dépôt: 08.03.1995
(51) Int. Cl.: C07D 335/06, C07D 339/08, C07C 217/62, C07C 217/60, C07D 327/06, C07D 319/08, C07D 319/18, C07D 317/66, C07D 311/58, C07D 239/74, C07D 241/42, C07D 237/28, C07D 217/02, C07D 215/26, C07D 307/79

(54) **Nouveaux composés alkylaminoindanes, leurs procédés de préparation et les compositions pharmaceutiques qui les contiennent**
Alkylaminoindanverbindungen, Verfahren zu deren Herstellung und diese enthaltenden pharmazeutische Zusammenstellungen
Alkylaminoindane compounds, processes for their preparation and pharmaceutical compositions containing them

(30) Priorité: 11.03.1994 FR 9402813
(43) Date de publication de la demande: 13.09.1995
(73) Titulaire: ADIR ET COMPAGNIE, 92415 Courbevoie Cédex (FR)
(72) Inventeur: Guillaumet, Gérald, F-45650 St. Jean le Blanc (FR); Viaud, Marie-Claude, F-45100 Orleans (FR); Renard, Pierre, F-78000 Versailles (FR); Adam, Gérard, F-78600 Le Mesnil le Roi (FR); Caignard, Daniel-Henri, F-75015 Paris (FR); Guardiola, Béatrice, F-92210 Saint-Cloud (FR); Rettori, Marie-Claire, F-92400 Courbevoie (FR)

(56) Documents cités:
- EP-A- 0 286 278
- EP-A- 0 558 245

## Description

La présente invention concerne de nouveaux dérivés alkylaminoindanes, leur procédé de préparation et les compositions pharmaceutiques qui les contiennent.

La demande de brevet EP 286 278 décrit déjà des dérivés indanamines de formule (A), 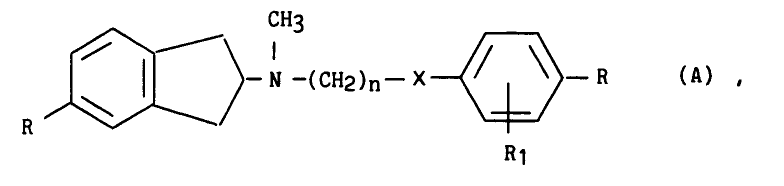 dans laquelle R, R₁, X et n sont tels que définis dans la demande EP 286 278, mais uniquement en tant qu'agents antiarythmiques.

La demanderesse a découvert de nouveaux dérivés alkylaminoindanes qui présentent de façon surprenante une très forte affinité pour les récepteurs sérotoninergiques. Ils possèdent notamment un intense pouvoir de liaison non seulement des récepteurs 5-HT_{1A}, mais également des récepteurs 5-HT_{2C} (anciennement dénommés récepteurs 5-HT_{1C}).

Ces caractéristiques les rendent utiles en thérapeutique, dans les troubles du système nerveux central (anxiété, dépression, stress, psychoses, schizophrénie, douleur, troubles du comportement alimentaire et sexuel et troubles du sommeil).

Plus spécifiquement, la présente invention a pour objet les composés de formule (I) : 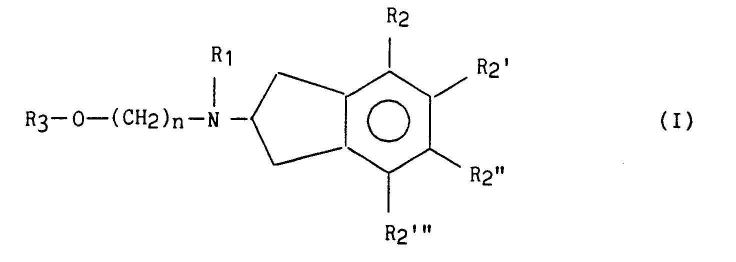 dans laquelle :
- R₁ représente un atome d'hydrogène ou un radical choisi parmi alkyle non substitué ou substitué, cycloalkyle, cycloalkylalkyle, phényl non substitué ou substitué et phénylalkyle non substituté ou substitué
- R₂, R₂', R₂'' et R₂''' représentent indépendamment l'un de l'autre un atome d'hydrogène, ou un radical choisi parmi halogène, alkyle non substitué ou substitué et alkoxy non substitué ou substitué,
- n représente un nombre entier de 1 à 6,
- R₃ représente un groupement de formule (A) : 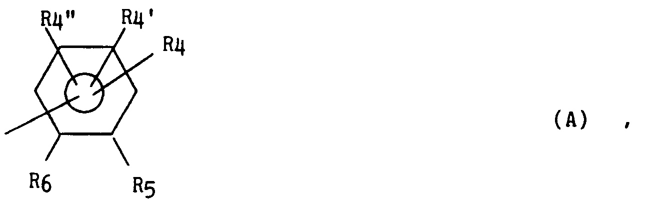 dans laquelle :
- R₄, R₄' et R₄'' représentent indépendemment l'un de l'autre un atome d'hydrogène ou un radical choisi parmi halogène, alkyle non substitué ou substitué et alkoxy inférieur non substitué ou substitué,
- R₅ et R₆ forment ensemble avec le noyau benzène qui les porte un système cyclique E₁ choisi parmi : indène, naphtalène, benzothiophène, benzofurane, indole, benzimidazole, benzopyrane, benzothiopyrane, chromane, thiochromane, quinoléine, isoquinoléine, indazole, 2,3-dihydro1,4-benzodithiine, quinoxaline, quinazoline, cinnoline, benzothiazole, benzisothiazole, benzoxazole, benzisoxazole, 2,3-dihydro1,4-benzoxathiine, 1,4-benzoxazine, 1,4-benzothiazine, 1,3-benzodioxole, 1,3-benzodioxane, 1,4-benzodioxane et 1,4-benzodioxine,
   étant entendu que la partie du système cyclique E₁ formé par R₅ et R₆ et les 2 atomes de carbone du noyau benzène qui les portent est :
- non hydrogénée ou partiellement hydrogénée,
- et non substituée ou substituée par un ou plusieurs radicaux choisis parmi : halogène, hydroxyle, alkyle inférieur, alkoxy non substitué ou substitué, alkoxycarbonyle inférieur, et carboxyle,
   étant entendu que, sauf précisions contraires :
   les termes "alkyle" et "alkoxy" signifient des groupements, linéaires ou ramifiés, contenant de 1 à 6 atomes de carbone,
   le terme "substitué" associé aux radicaux "alkyle" et "alkoxy" signifie "substitué par un ou plusieurs radicaux choisis parmi halogène, hydroxyle et alkoxy",
   le terme "cycloalkyle" désigne un groupement cyclique de 3 à 8 atomes de carbone,
   le terme "substitué" associé aux radicaux "phényle" et "phénylalkyle" signifie que ces radicaux peuvent être substitué sur le noyau phényle par un ou plusieurs substituants choisis parmi halogène, alkyle, alkoxy, hydroxyle et polyhalogénoalkyl,
   leurs isomères optiques, ainsi que leurs sels d'addition à un acide ou une base pharmaceutiquement acceptable.

De façon particulière, les radicaux alkyles présents dans les différents substituants de la formule (I) peuvent être choisis parmi méthyle, éthyle, n-propyle, isopropyle, n-butyle, isobutyle, sec-butyle, tert-butyle, pentyle et hexyle.

Les radicaux alkoxy présents dans les substituants de la formule (I) peuvent être choisis parmi méthoxy, éthoxy, n-propoxy, isopropoxy, n-butoxy, iso-butoxy, sec-butoxy, tert-butoxy, pentyloxy, et hexyloxy.

Les halogènes présents dans les substituants de la formule (I) peuvent être choisis parmi le brome, le chlore, le fluor, et l'iode.

Les cycloalkyles présents dans les substituants de la formule (I) peuvent être choisis parmi cylclopropyle, cyclobutyle, cyclopentyle, cyclohexyle, cycloheptyle et cyclooctyle.

Les groupements -(CH2)n- présents dans la formule (I) peuvent être choisis parmi méthylène, éthylène, triméthylène, tétraméthylène, pentaméthylène et hexaméthylène.

Parmi les acides pharmaceutiquement acceptables que l'on peut utiliser pour former un sel d'addition avec les composés de l'invention , on peut citer, à titre d'exemples et de façon non limitative, les acides chlorhdrique, sulfurique, phosphorique, tartrique, malique, maléique, fumarique, oxalique, méthanesulfonique, éthanesulfonique, camphorique, et citrique.

Parmi les bases pharmaceutiquement acceptables que l'on peut utiliser pour salifier les composés utilisés selon l'invention, on peut citer, à titre d'exemples et de façon non limitative la soude, la potasse, la triéthylamine, la diéthylamine, l'éthanolamine, l'arginine, la lysine, et la diéthanolamine.

L'invention concerne plus particulièrement les composés de formule (I) dans lesquels R₅ et R₆ forment ensemble avec le noyau benzène qui les porte un système cyclique E₁ choisi parmi naphthalène, benzopyrane, benzothiopyrane, chromane, thiochromane et benzodioxane.

Par exemple, l'invention concerne les composés de formule (I) dans lesquels R₅ et R₆ forment ensemble avec le noyau benzène qui les porte un système cyclique E₁ choisi parmi thiochromane, chromane et benzodioxane.

L'invention s'étend également au procédé de préparation des composés de formule (I), caractérisé en ce que l'on fait réagir un dérivé hydroxylé de formule (II) : 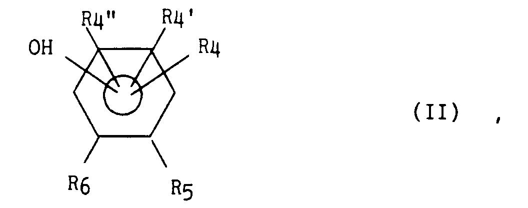 dans laquelle R₄, R₄', R₄'', R₅ et R₆ sont tels que définis dans la formule (I),
avec un dihalogénoalkyle de formule (III) :

X-(CH₂)ₙ-X', (III)

dans laquelle X et X' sont des atomes d'halogène et n est tel que défini dans la formule (I), pour donner un composé de formule (IV) : 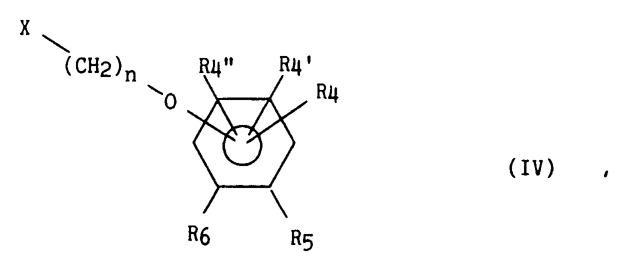 dans laquelle n, X, R₄, R₄', R₄'', R₅, et R₆ sont tels que définis précédemment,
le dit composé de formule (IV) est mis en réaction avec un aminoindane de formule (V) : 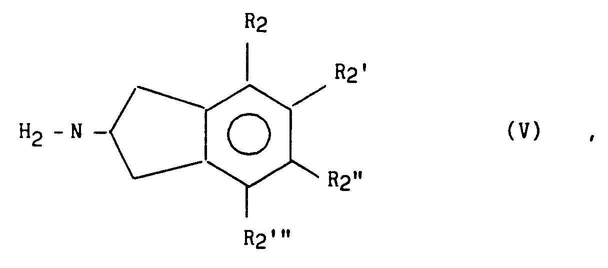 dans laquelle R₂, R₂', R₂'' et R₂''' sont tels que définis dans la formule (I), pour donner un composé de formule (Ia) : 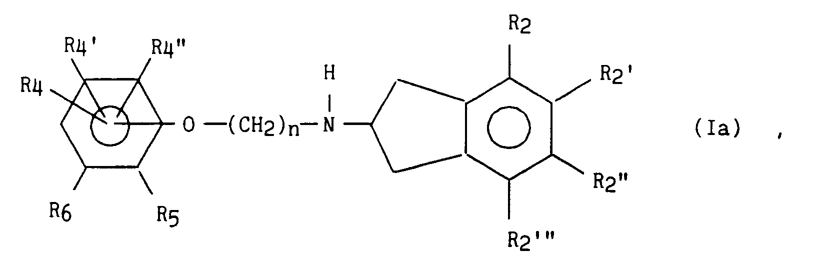 dans laquelle R₂, R₂', R₂'', R₂''', R₄, R₄', R₄'', R₅, R₆ et n sont tels que définis précédemment,
le dit composé de formule (Ia) pouvant être mis en présence d'un halogénoalkyle de formule (VI) :

R₁'-X'', (VI)

dans laquelle X'' est un atome d'halogène et R₁' a la même signification que R₁, tel que défini dans la formule (I) à l'exception de l'hydrogène, pour donner un composé de formule (Ib) : 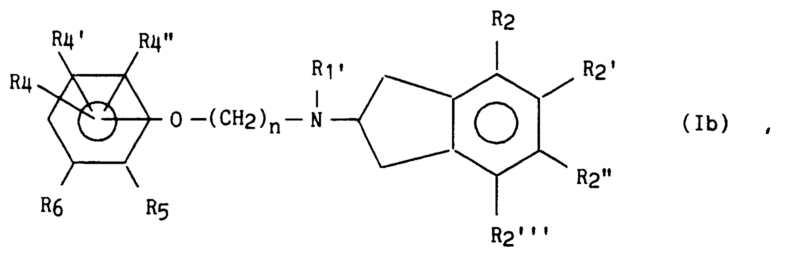 dans laquelle R₁', R₂, R₂', R₂'', R₂''', R₄, R₄', R₄'', R₅, R₆ et n sont tels que définis précédemment,
les composés de formule (Ia) et (Ib) constituant l'ensemble des composés de formule (I) qui peuvent être, si on le désire,
- purifiés suivant une ou plusieurs méthodes de purification choisies parmi la cristallisation, la chromatographie sur colonne de silice, l'extraction, la filtration, et le passage sur charbon et/ou résine,
- séparés, le cas échéant, sous forme pure ou sous forme de mélange, en leurs éventuels isomères optiques,
- et/ou salifiés par un acide ou une base pharmaceutiquement acceptable.

L'invention s'étend aux variantes du procédé ci-dessus décrit, étant entendu qu'elles soient réalisables par l'homme du métier.

Les matières premières utilisées lors du procédé précédemment décrit sont soit disponibles dans le commerce, soit aisément accessibles à l'homme du métier d'après la littérature.

La demanderesse a découvert que les composés de l'invention possédaient des propriétés pharmacologiques très intéressantes.

Les composés de l'invention présentent de façon surprenante une très forte affinité pour les récepteurs sérotoninergiques et notamment un intense pouvoir antagoniste des récepteurs 5-HT_{1A} et 5-HT_{2C}.

Des essais de liaison aux récepteurs 5-HT_{1A} et 5-HT_{1C} ont en effet montré que les composés de l'invention se comportent comme de puissants ligands des récepteurs sérotoninergiques 5-HT_{1A} et 5-HT_{1C} (exemple B de la présente demande).

L'activité antagoniste des composés de l'invention a été mise en évidence in-vitro et se traduit in-vivo par une très forte activité anxiolytique (test dit des cages claires/obscures chez la souris, exemple F de la présente demande) associée à une remarquable activité antidépressive (test sur les échecs d'échappement chez le rat, exemple E de la présente demande).

De ce fait, les composés de formule générale (I) et leurs sels physiologiquement tolérables possèdent des propriétés pharmacologiques et thérapeutiques intéressantes, notamment des propriétés anxiolytiques antidépressives et régulatrices des troubles du sommeil.

Les composés de la présente invention peuvent ainsi être utilisés dans le traitement et la prévention des troubles liés aux récepteurs 5-HT_{1A} ou 5-HT_{2C}. Par exemple, ils peuvent être utiles dans le traitement et la prévention du stress, de l'anxiété, de la dépression, des psychoses, de la schizophrénie, de la douleur, des troubles du comportement alimentaire et sexuel et des troubles du sommeil.

D'autre part, les composés de l'invention potentialisent de façon surprenante les effets des anti-dépresseurs connus et permettent leur efficacité immédiate (disparition de la latence d'une quinzaine de jour généralement observée).

La présente invention a également pour objet les compositions pharmaceutiques contenant un composé de formule (I), ou un de ses sels physiologiquement tolérables, en combinaison avec un ou plusieurs excipients ou véhicules pharmaceutiquement acceptables.

Parmi les compositions pharmaceutiques selon l'invention, on pourra citer, à titre d'exemples et de façon non limitative, celles qui conviennent à une administration par voie orale, rectale, nasale ou parentérale et notamment les comprimés, les dragées, les gélules, les paquets, les sachets, les granules, les pilules, les granulés, les suppositoires, les crèmes, les pommades, les aérosols, les capsules, les gels dermiques et les solutions injectables ou buvables.

La posologie varie d'un individu à l'autre, selon l'âge, le poids, et le sexe du malade, la voie d'administration choisie, la nature et l'intensité de l'affection. Les doses utilisées s'échelonnent entre 0,1 et 100 mg par jour, divisibles en 1 à 3 prises par 24 h, plus particulièrement 1 à 10 mg par jour, par exemple 5 mg par jour.

Les exemples suivants illustrent l'invention et ne la limitent en aucune façon.

### EXEMPLE 1 : 8-{4-[(N-INDAN-2-YL)AMINO] BUTYLOXY}THIOCHROMANE

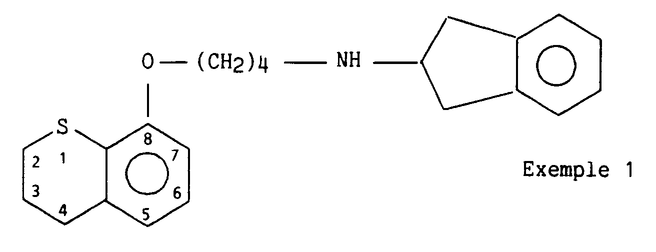

### Stade A : 8-(4-bromobutanoxy)thiochromane

Dissoudre 300 mg de 8-hydroxythiochromane (tel que décrit dans le brevet EP 571243) dans 3 cm³ de N,N-diméthylformamide sous argon. On ajoute 430 mg (1,985 mmoles) de 1,4-dibromobutane dilués dans 3 cm³ de N, N-diméthylformamide.
Ajouter ensuite 750 mg de carbonate de potassium à la spatule.
Porter à 65°C sous argon et sous agitation pendant 3h et 30 mn.
Evaporer le N,N-diméthylfornamide. Reprendre le résidu par H₂O et extraire au dichlorométhane, avant de le purifier sur une colonne de silice (éluant : éther/ éther de pétrole ; 15 : 85).
On récupère 240 mg de cristaux blancs de 8-(4-bromobutanoxy)thiochromane.

### Stade B : 8-{4-[(N-indan-2-yl)amino]butyloxy}thiochromane

Sous argon, dissoudre lg (3,32 mmol) de dérivé bromé préparé au stade A, 850 mg (4,98 mmol) de chlorhydrate de 2-aminoindane et 2,15 g (16,60 mmol) de N,N diisopropyléthylamine dans 40 cm³ d'acétonitrile. Maintenir à reflux pendant 40 heures.
Evaporer le solvant. Reprendre le résidu par de l'eau et extraire au dichlorométhane. Sécher la phase organique sur sulfate de magnésium.
Après concentration, purifier sur colone de silice normale (éluant méthanol/dichlorométhane 5:95). On récupère 630 mg d'un solide très coloré qu'on recristallise dans l'éthanol.
Rendement : 54 %
Point de fusion : 179-180°C
   - IR (KBr)cm⁻¹: : 3600-3200 (v NH)
   1255 (v C-0)
   - SM (m/e): : 354 (M+1)
¹H RMN 300MHz (CDCl₃) :
   1,70(s,1H,NH) ; 1,85-2,15 (m,6H,CH₂);275(t,2H J=59Hz CH₂Ar₁);2,83-2,89 (m, 2H,CH₂S) 3,07(t,2H,J=7,4Hz,CH2-N) ; 3,23(dd,2H,J₁=15,4Hz,J₂=7,4Hz, 2xAr₂CHCHN) 3,33(dd,2H,J₁:15,4Hz,J₂:7,4Hz,2xAr₂CHCHN) ;3,93(quintuplet, 1H,J=7,4Hz,CHN) ; 3,99 (t,2H,J=5,9Hz,O-CH2) ; 6,58(d, 1H,J=7,4Hz, Arom); 6,63(d, lH,J=7,4Hz,Arom) ; 6,89(t,1H,J=7,4Hz,Arom) ; 7,09-7,18(m,4H,Arom).

### EXEMPLE 2 : 8-{3-[(N-INDAN-2-YL)AMINO]PROPYLOXY}THIOCHROMANE

### Stade A : 8-(3-bromopropyloxy)thiochromane

En procédant de la même façon qu'au stade A de l'exemple 1, mais en remplaçant le 1,4 dibromobutane par le 1,3 dibromopropane, on obtient le composé du titre.

### Stade B : 8-{3-[(N-indan-2-yl)amino]propyloxy}thiochromane

Sous argon, dissoudre 1 g (3,48 mmol) de dérivé bromé préparé au stade A, 890 mg (5,22 mmol) de chlorhydrate de 2-aminoindane et 2,25 g (17,41 mmol) de N,N diisopropyléthylamine dans 35 cm³ d'acétonitrile.
Maintenir à reflux pendant 40 heures.
Evaporer le solvant. Reprendre le résidu par H₂O puis extraire au dichlorométhane. Sécher sur sulfate de magnésium. Après concentration, purifier sur colonne de silice normale (éluant méthanol/dichlorométhane 5:95).
On récupère 740 mg d'une huile très colorée dont on fait le fumarate.
Rendement : 63 %
Point de fusion (fumarate) : 170°C
   - IR (film)cm⁻¹: : 3600-3200 (v NH)
   1250 (v C-0)
   - SM (m/e): : 340 (M+1)
¹H RMN 300MHz (CDCl₃) :
   1,70(s,1H,NH) ; 1,96-2,11 (m,4H,CH₂) ; 2,73-2,99 (m, 8H, CH₂Ar₁,CH₂S,CH₂N, 2xAr₂CHCHN) ;3,16(dd,2H,J₁=15,4Hz,J₂=6,6Hz,2xAr₂CHCHN); 3,64(quintuplet, 1H,J=6,6Hz,CHN) ; 4,08 (t,2H,J=5,9Hz,O-CH₂) ; 6,60-6,68 (m,2H,Arom) ; 6,90(t,1H,J=7,4Hz,Arom) ; 7,08-7,20(m,4H,Arom).

### EXEMPLE 3 : 8-{2-[(N-INDAN-2-YL)AMINO]ETHOXY}THIOCHROMANE

### Stade A : 8-(2-bromoéthoxy)thiochromane

En procédant de la même façon qu'au stade A de l'exemple 1, mais en remplaçant le 1,4-dibromobutane par le 1,2-dibromoéthane, on obtient le composé du titre.

### Stade B : 8-{2-[(N-indan-2-yl)amino]éthoxy}thiochromane

Sous argon, dissoudre 500 mg (1,83 mmol) de dérivé bromé préparé au stade A, 470 mg (2,75 mmol) de chlorhydrate de 2-aminoindane et 1,2g (9,15 mmol) de N,N-diisopropyléthylamine dans 15 cm³ d'acétonitrile. Maintenir à reflux pendant 40 heures.
Evaporer le solvant. Reprendre le résidu par H₂O et extraire au dichlorométhane. Sécher la phase organique sur sulfate de magnésium. Après concentration, purifier sur colonne de silice normale (éluant : méthanol/dichlorométhane ; 1:99). On récupère 310 mg d'un solide très coloré.
Rendement : 52 %
Point de fusion (fumarate) : 173°C
   - IR (KBr)cm⁻¹: : 3600-3200 (v NH)
   1250 (v C-0)
   - SM (m/e): : 326 (M+1)
¹H RMN 300MHz (CDCl₃) :
   1,70(s,1H,NH) ; 2,02-2,13(m,2H,CH₂); 2,77-2,88(m,4H, CH₂Ar,2xAr₂CHCHN) ; 2,94-3,02(m,2H,CH₂S) ; 3,08(t,2H,J=5,2Hz,CH₂N) ; 3,21 (dd,2H,J₁=15,5 Hz, J₂=6,9Hz,2xAr₂CHCHN) ; 3,71(quintuplet,1H,J=6,9Hz,CHN) ; 4,14(t,2H, J=5,2Hz,CH-O) ; 6,63-6,71 (m,2H,Arom) ; 6,92(t,1H,J=7,7Hz,Arom) ; 7,09-7,23(m,4H,Arom).

### EXEMPLE 4 : 8-{3-[(N-PROPYL N-INDAN-2-YL)AMINO]PROPYLOXY}THIOCHROMANE

Sous argon, dissoudre 180 mg (5,30.10⁻⁴ mol) de 8-{3-[(N-indan-2-yl)amino]propyloxy}thiochromane préparé selon l'exemple 2, 540 mg (3,18 mmol) de 1-iodopropane et 200 mg (1,59 mmol) de N,N-diisopropyléthylamine dans 2 cm³ de diméthylformamide. Maintenir à 60°C pendant 24 heures puis évaporer le solvant. Reprendre le résidu par de l'eau et extraire au dichlorométhane. Sécher la phase organique sur sulfate de magnésium. Après concentration, purifier sur colonne de silice normale (éluant : Acétate d'éthyl 100%). On obtient 170 mg d'un solide beige que l'on recristallise dans le cyclohexane.
Rendement : 84 %
Point de fusion : 68-69°C
   - IR (KBr)cm⁻¹: : 1260 (v C-0)
   - SM (m/e): : 382 (M+1)
¹1H RMN 300MHz (CDCl₃) :
   0,89(t,3H,J:7,3Hz,CH₃) ; 1,46-1,61(m,2H,CH₂) ; 1,92-2,13(m,4H,CH₂) ; 2,49-2,57(m,2H,CH₂) ; 2,76-3,09(m, 10H, CH₂) ; 3,68(quintuplet,1H,J=7,9Hz,CH-N) ; 4,06(t,2H,J=6,1Hz,CH₂-O) ; 6,62-6,68 (m,2H,Arom) ; 6,92(t,1H,J=7,9Hz,Arom) ; 7,09-7,19(m,4H, Arom).

### EXEMPLE 5 à 8 :

En procédant comme dans les exemples 1 à 4, mais en remplaçant le 8-thiochromanol par le 1-hydroxynaphtalène (tel que décrit dans le J. Labelled Compd. Radiopharm.;85;vol.22(11);pp.1149-54), on obtient les composés suivants :

### EXEMPLE 5 : 1-{4-[(N-INDAN-2-YL)AMINO]BUTYLOXY}NAPHTALENE

### EXEMPLE 6 : 1-{3-[(N-INDAN-2-YL)AMINO]PROPYLOXY}NAPHTALENE

### EXEMPLE 7 : 1-{2-[(N-INDAN-2-YL)AMINO]ETHOXY}NAPHTALENE

### EXEMPLE 8 : 1-{3-[(N-PROPYL N-INDAN-2-YL)AMINO]PROPYLOXY}NAPHTALENE

### EXEMPLES 9 à 12 :

En procédant comme dans les exemples 1 à 4, mais en remplaçant le 8-thiochromanol par le 8-hydroxyquinoléine (tel que décrit dans J. Mater. Chem.;91;vol.1(3);pp.327-30), on obtient les composés suivants :

### EXEMPLE 9 : 8-{4-[(N-INDAN-2-YL)AMINO]BUTYLOXY}QUINOLEINE

### EXEMPLE 10 : 8-{3-N-INDAN-2-YL)AMINO]PROPYLOXY}QUINOLEINE

### EXEMPLE 11 : 8-{2-N-INDAN-2-YL)AMINO]ETHOXY}QUINOLEINE

### EXEMPLE 12 : 8-{3-[(N-PROPYL N-INDAN-2-YL)AMINO]PROPYLOXY}QUINOLEINE

### EXEMPLE 13 à 16 :

En procédant comme dans les exemples 1 à 4, mais en remplaçant le 8-hydroxythiochromane par le 8-hydroxy 2-H benzopyrane (tel que décrit dans Chem. Pharm. Bull.;86;vol.34(5);pp 2024-36), on obtient les composés suivants :

### EXEMPLE 13 : 8-{4-[(N-INDAN-2-YL)AMINO]BUTYLOXY}-2H-BENZOPYRANE

### EXEMPLE 14 : 8-{3-[(N-INDAN-2-YL)AMINO]PROPYLOXY}-2H-BENZOPYRANE

### EXEMPLE 15 : 8-{2-[(N-INDAN-2-YL)AMINO]ETHOXY}-2H-BENZOPYRANE

### EXEMPLE 16 : 8-{3-[(N-PROPYL N-INDAN-2-YL)AMINO]PROPYLOXY}-2H-BENZOPYRANE

### EXEMPLES 17 à 20 :

En procédant comme dans les exemples 1 à 4 mais en remplaçant le 8-hydroxythiochromane par le 8-hydroxychromane (tel que décrit dans Chem. Pharm. Bull.;87;vol. 35(2);pp. 632-41), on obtient les composés suivants :

### EXEMPLE 17 : 8-{4-[(N-INDAN-2-YL)AMINO]BUTYLOXY}CHROMANE

### EXEMPLE 18 : 8-{3-[(N-INDAN-2-YL)AMINO]PROPYLOXY}CHROMANE

### EXEMPLE 19 : 8-{2-[(N-INDAN-2-YL)AMINO]ETHOXY}CHROMANE

Point de fusion : 170-172°C

### EXEMPLE 20 : 8-{3-[(N-PROPYL N-INDAN-2-YL)AMINO]PROPYLOXY}CHROMANE

### EXEMPLE 21 à 24 :

En procédant comme dans les exemples 1 à 4, mais en remplaçant le 8-hydroxythiochromane par la 8-hydroxy-2,3,4-tetrahydro isoquinoléine (tel que décrit dans J. Med. Chem.;87;vol.30(112);pp.2208-16), on obtient les composés suivants :

### EXEMPLE 21 : 8-{4-[(N-INDAN-2-YL)AMINO]BUTYLOXY}1,2,3,4-TETRAHYDRO-ISOQUINOLEINE

### EXEMPLE 22 : 8-{3-[(N-INDAN-2-YL)AMINO]PROPYLOXY}1,2,3,4-TETRAHYDRO-ISOQUINOLEINE

### EXEMPLE 23 : 8-{2-[(N-INDAN-2-YL)AMINO]ETHOXY}1,2,3,4-TETRAHYDRO-ISOQUINOLEINE

### EXEMPLE 24 : 8-{3-[(N-PROPYL N-INDAN-2-YL)AMINO]PROPYLOXY} 1,2,3,4-TETRAHYDRO-ISOQUINOLEINE

### EXEMPLES 25 à 28 :

En procédant comme dans les exemples 1 à 4, mais en remplaçant le 8-hydroxythiochromane par la 5-hydroxyquinoxaline (tel que décrit dans Recl. Trav. Chim. Pays-Bas;76;vol. 95 (12);pp.285-9), on obtient les composés suivants :

### EXEMPLE 25 : 5-{4-[(N-INDAN-2-YL)AMINO]BUTYLOXY}QUINOXALINE

### EXEMPLE 26 : 5-{3-[(N-INDAN-2-YL)AMINO]PROPYLOXY}QUINOXALINE

### EXEMPLE 27 : 5-{2-[(N-INDAN-2-YL)AMINO]ETHOXY}QUINOXALINE

### EXEMPLE 28 : 5-{3-[(N-PROPYL N-INDAN-2-YL)AMINO]PROPYLOXY}QUINOXALINE

### EXEMPLES 29 à 32 :

En procédant comme dans les exemples 1 à 4, mais en remplaçant le 8-hydroxythiochromane par la 8-hydroxyquinazoline (tel que décrit dans Text. Chem. Color.;92;vol.24(9);pp.66-71) on obtient les composés suivants :

### EXEMPLE 29 : 8-{4-[(N-INDAN-2-YL)AMINO]BUTYLOXY}QUINAZOLINE

### EXEMPLE 30 : 8-{3-[(N-INDAN-2-YL)AMINO]PROPYLOXY}QUINAZOLINE

### EXEMPLE 31 : 8-{2-[(N-INDAN-2-YL)AMINO]ETHOXY}QUINAZOLINE

### EXEMPLE 32 : 8-{3-[(N-PROPYL N-INDAN-2-YL)AMINO]PROPYLOXY}QUINAZOLINE

### EXEMPLE 33 à 36 :

En procédant comme dans les exemples 1 à 4, mais en remplaçant le 8-hydroxythiochromane par la 8-hydroxycinnoline (tel que décrit dans Tetrahedron;78;vol.34(7) ; pp.941-6), on obtient les composés suivants :

### EXEMPLE 33 : 8-{4-[(N-INDAN-2-YL)AMINO]BUTYLOXY}CINNOLINE

### EXEMPLE 34 : 8-{3-[(N-INDAN-2-YL)AMINO]PROPYLOXY}CINNOLINE

### EXEMPLE 35 : 8-{2-[(N-INDAN-2-YL)AMINO]ETHOXY}CINNOLINE

### EXEMPLE 36 : 5-{4-[(N-PROPYL N-INDAN-2-YL)AMINO]BUTYLOXY}CINNOLINE

### EXEMPLES 37 à 40 :

En procédant comme dans les exemples 1 à 4, mais en remplaçant le 8-hydroxythiochromane par la 8-hydroxy 1,3-benzodioxane (tel que décrit dans la demande de brevet EP 103173), on obtient les composés suivants :

### EXEMPLE 37 : 8-{4-[(N-INDAN-2-YL)AMINO]BUTYLOXY}-1,3-BENZODIOXANE

### EXEMPLE 38 : 8-{3-[(N-INDAN-2-YL)AMINO]PROPYLOXY}-1,3-BENZODIOXANE

### EXEMPLE 39 : 8-{2-[(N-INDAN-2-YL)AMINO]ETHOXY}-1,3-BENZODIOXANE

### EXEMPLE 40 : 8-{4-[(N-PROPYL N-INDAN-2-YL)AMINO]BUTYLOXY}-1,3-BENZODIOXANE

### EXEMPLE 41 à 45 :

En procédant comme dans les exemples 1 à 4, mais en remplaçant le 8-hydroxythiochromane par le 7-hydroxy 1-H indène (tel que décrit dans J. Am. Chem. Soc.;93;vol.115(17);pp.7653-64), on obtient les composés suivants :

### EXEMPLE 41 : 7-{4-[(N-INDAN-2-YL)AMINO]BUTYLOXY}INDENE

### EXEMPLE 42 : 7-{3-[(N-INDAN-2-YL)AMINO]PROPYLOXY}INDENE

### EXEMPLE 43 : 7-{2-[(N-INDAN-2-YL)AMINO]ETHOXY}INDENE

### EXEMPLE 44 : 7-{4-[(N-PROPYL N-INDAN-2-YL)AMINO]BUTYLOXY}INDENE

### EXEMPLES 45 à 48 :

En procédant comme dans les exemples 1 à 4, mais en remplaçant le 8-hydroxythiochromane par la 4-hydroxy 1,3-benzodioxole (tel que décrit dans J. Chem. Soc., Faraday Trans.2;79;vol.75(12);pp.1637-42), on obtient les composés suivants :

### EXEMPLE 45 : 4-{4-[(N-INDAN-2-YL)AMINO]BUTYLOXY}I1,3-BENZODIOXOLE

### EXEMPLE 46 : 4-{3-[(N-INDAN-2-YL)AMINO]PROPYLOXY}1,3-BENZODIOXOLE

### EXEMPLE 47 : 4-{2-[(N-INDAN-2-YL)AMINO]ETHYLOXY}1,3-BENZODIOXOLE

### EXEMPLE 48 : [(N-PROPYL N-INDAN-2-YL) 4-{4-AMINO]BUTYLOXY}1,3-BENZODIOXOLE

### EXEMPLES 49 à 52 :

### En procédant comme dans les exemples 1 à 4, mais en remplaçant le 8-hydroxythiochromane par le 4-hydroxy 2,3-dihydrobenzofurane (tel que décrit dans Hétérocycles;92;vo.34(7);pp.1353-64), on obtient les composés suivants :

### EXEMPLE 49 : 7-{4-[(N-INDAN-2-YL)AMINO]BUTYLOXY}BENZOFURANE

### EXEMPLE 50 : 7-{3-[(N-INDAN-2-YL)AMINO]PROPYLOXY}BENZOFURANE

### EXEMPLE 51 : 7-{2-[(N-INDAN-2-YL)AMINO]ETHOXY}BENZOFURANE

### EXEMPLE 52 : 7-{4-[(N-PROPYL N-INDAN-2-YL)AMINO]BUTYLOXY}BENZOFURANE

### EXEMPLES 53 à 56 :

En procédant comme dans les exemples 1 à 4, mais en remplaçant le 8-hydroxythiochromane par le 5-hydroxy 2,3-dihydro 1,4-benzoxathiine (tel que décrit dans le brevet US 3,636,047), on obtient les composés suivants :

### EXEMPLES 53 : 5-{4-[(N-INDAN-2-YL)AMINO]BUTYLOXY}-2,3-DIHYDRO-1,4-BENZOXATHIINE

### EXEMPLE 54 : 5-{3-[(N-INDAN-2-YL)AMINO]PROPYLOXY}-2,3-DIHYDRO-1,4-BENZOXATHIINE

### EXEMPLE 55 : 5-{2-[(N-INDAN-2-YL)AMINO]ETHOXY}-2,3-DIHYDRO-1,4-BENZOXATHIINE

### EXEMPLE 56 : 5-{3-[(N-PROPYL N-INDAN-2-YL)AMINO]PROPYLOXY}-2,3-DIHYDRO-1,4-BENZOXATHIINE

### EXEMPLES 57 à 60 :

En procédant comme dans les exemples 1 à 4, mais en remplaçant le 8-hydroxythiochromane par le 5-hydroxy 2,3-dihydro 1,4-benzodithiine (tel que décrit dans le brevet US 3,636,047), on obtient les composés suivants :

### EXEMPLES 57 : 5-{4-[(N-INDAN-2-YL)AMINO]BUTYLOXY}-2,3-DIHYDRO-1,4-BENZODITHIINE

### EXEMPLE 58 : 5-{3-[(N-INDAN-2-YL)AMINO]PROPYLOXY}-2,3-DIHYDRO-1,4-BENZODITHIINE

### EXEMPLE 59 : 5-{2-[(N-INDAN-2-YL)AMINO]ETHOXY}-2,3-DIHYDRO-1,4-BENZODITHIINE

### EXEMPLE 60 : 5-{3-[(N-PROPYL N-INDAN-2-YL)AMINO]PROPYLOXY}-2,3-DIHYDRO-1,4-BENZODITHIINE

### EXEMPLE 61 À 64 :

En procédant comme dans les exemples 1, 2 et 4 (pour l'exemple 64), mais en remplaçant le 8-hydroxythiochromane par le 5-hydroxy 1,4-benzodioxane, on obtient les composés suivants :

### EXEMPLE 61 : 5-{4-[(N-INDAN-2-YL)AMINO]BUTYLOXY}-1,4-BENZODIOXANE

### EXEMPLE 62 : 5-{3-[(N-INDAN-2-YL)AMINO]PROPYLOXY}-1,4-BENZODIOXANE

### EXEMPLE 63 : 5-{2-[(N-INDAN-2-YL)AMINO]ETHOXY}-1,4-BENZODIOXANE

### Stade A : 5-METHOXY 1,4-BENZODIOXANE

Un mélange de dibromoéthane (10,73 g, 77 mmol) et de bromure de cétyltriméthylammonium (0,291 g, 0,8 mmol) dans 5 cm³ d'eau est porté à reflux. Une solution de 3-méthoxycatéchol (5 g, 35,7 mmol) dans la soude 20N (NaOH, 4 g dans 5 cm³ d'H₂O) y est additionnée lentement. Le mélange est agité à reflux pendant 12 heures puis, après refroidissement, extraction à l'éther, la purification sur colonne de silice (éluant : éther de pétrole / éther 9 : 1) permet d'isoler 7,4 g d'une huile incolore.
Rendement : 80 %

### Stade B 5-HYDROXY BENZODIOXANE

A 0°C, le tribromure de bore (5,6 cm³) est additionné lentement à une solution de 5-méthoxy 1,4-benzodioxane (5 g, 30,1 mmol) dans 25 cm³ de CH₂Cl₂. Le mélange est agité à 0°C pendant 15 mn puis la solution est hydrolysée par 10 cm³ d'eau et le produit est extrait au CH₂Cl₂. La purification sur colonne de silice (éluant CH₂Cl₂ 100 %) permet d'obtenir 4,49 g d'une huile incolore.
Rendement : 95 %

### Stade C : 5-[3-BROMOETHYLOXY]BENZODIOXANE

Mettre en solution 730 mg (4,80 mmol) du 5-hydroxy benzodioxane et 1,80 g (9,60 mmol, 0,82 cm³) de 1,2-dibromoéthane à 100°C. Refroidir puis additionner goutte à goutte 4,5 cm³ (7,20 mmol) d'une solution aqueuse de soude 1,6 N. Après 24 heures à 100°C, refroidir et reprendre par 35 cm³ de soude aqueuse 2N. Le produit est extrait au CH₂Cl₂ puis purifié sur colonne de silice (éluant CH₂Cl₂ 100%). On récupère 700 mg d'un solide blanc.
Point de fusion : 86°C
Rendement : 56 %

### Stade D : 5-{2-[(N-INDAN-2-YL)AMINO]ETHOXY}1,4-BENZODIOXANE

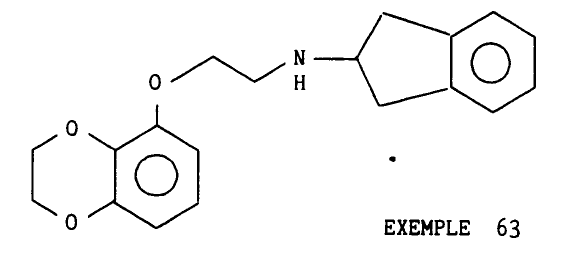

Dissoudre 50 mg (1,93 x 10-4 mol) de dérivé bromé obtenu au stade précédent, 40 mg (2,89 x 10-4 mol) de chlorydrate de 2-aminoindane et 125 mg (9,65 x 10-4 mol) de N,N-diisopropyléthylamine dans 2,5 cm³ d'acétonitrile. Après 40 heures à reflux, évaporer le solvant. Reprendre le résidu par H₂O et extraire au CH₂Cl₂. Après purification sur colonne de silice (éluant CH₂Cl₂ 100 % → MeOH/CH₂Cl₂ 1 : 99), on obtient 40 mg d'une huile très colorée dont on fait le fumarate.
Rendement : 66 %.

### EXEMPLE 64 : 5-{3-[(N-PROPYL N-INDAN-2-YL)AMINO]PROPYLOXY}1,4-BENZODIOXANE

### EXEMPLE 65 : 8-{3-[(N-METHYL N-INDAN-2-YL)AMINO]PROPYLOXY}THIOCHROMANE

Sous argon, dissoudre 1 g (2,95 mmol) de 8-{3-[(N-indan-2-yl)amino]propyloxy)thiochromane préparé selon l'exemple 2, 0,67 cm³ (740 mg, 11,78 mmol) d'acide acétique glacial et 740 mg (11,78 mmol) de cyanoborohydrure de sodium dans 15 cm³ de méthanol anhydre.
Refroidir dans un bain de glace.
A l'aide d'une ampoule à brome, additionner goutte à goutte 0,96 cm³ (354 mg, 11,78 mmol) de formaldéhyde à 37 % dans l'eau en solution dans 15 cm³ de méthanol.
Laisser sous agitation 3 heures à température ambiante puis additionner 10 cm³ d'une solution saturée de K₂CO₃. Chasser sous vide le méthanol. Reprendre par H₂O et extraire à l'ACOEt.
Après purification sur colonne de silice normale (éluant CH₂Cl₂ 100% →MeOH / CH₂Cl₂ 5:95), on obtient 940 mg d'un solide que l'on recristallise dans l'isopropanol.
Rendement 90%.
Point de fusion : 75-76 °C

### EXEMPLE 66 : 8-{2-[(N-PROPYL N-INDAN-2-YL)AMINO]ETHOXY}THIOCHROMANE

Point de fusion : 164°C

### ETUDE PHARMACOLOGIQUE DES COMPOSES DE L'INVENTION

### EXEMPLE A : ETUDE DE LA TOXICITE AIGUE

La toxicité aiguë a été effectuée après administration orale à des lots de cinq souris (20 ± 2 grammes) de doses croissantes (0,1 - 0,25 - 0,50 - 0,75 - 1g.kg⁻¹) des produits de l'invention.
Les animaux ont été observés à intervalles réguliers au cours de la première journée et quotidiennement pendant les deux semaines suivant le traitement. Il apparaît que les composés de l'invention sont totalement atoxiques aux doses testées. Aucun décès n'est observé après administration d'une dose de 1 g.kg⁻¹. On ne constate pas de troubles après administration de cette dose.

### EXEMPLE B : MESURE DE L'AFFINITE DES COMPOSES DE L'INVENTION POUR LES RECEPTEURS 5-HT_{1A}.

### PROTOCOLE :

L'affinité in-vitro des composés de l'invention, pour les récepteurs sérotoninergiques 5-HT_{1A}, a été déterminée par la mesure du déplacement de la [³H] 8-hydroxy-2-(di-n-propylamino)tétraline [ou [³H] 8-OH-DPAT], agoniste sélectif de ce récepteur, sur des préparations d'hippocampe de rat.

### RESULTATS :

Les composés de formule générale (I) se révèlent être de très puissants ligands des récepteurs 5-HT_{1A}, avec des constantes d'affinité de l'ordre du nanomolaire.

### EXEMPLE C : MESURE DE L'AFFINITE DES COMPOSES DE L'INVENTION POUR LES RECEPTEURS β₁, β₂, D₁, D₂, 5-HT_{1C}, 5-HT_{1D}, 5-HT₂ET 5-HT₃.

### PROTOCOLE :

L'affinité in-vitro des composés de l'invention a été déterminée :
- pour les récepteurs adrénergiques β₁, par la mesure du déplacement du dihydroalprénolol, sur des préparations de cortex frontal de rat,
- pour les récepteurs adrénergiques β₂, par la mesure du déplacement du dihydroalprénolol, sur des préparations de parenchyme pulmonaire de rat,
- pour les récepteurs dopaminergiques D₁, par la mesure du déplacement du SCH 23390, sur des préparations de striatum de rat,
- pour les récepteurs dopaminergiques D₂, par la mesure du déplacement du Raclopride, sur des préparations de striatum de rat,
- pour les récepteurs sérotoninergiques 5-HT_{1C}, par la mesure du déplacement du N-méthylmésulergine, sur des préparations de cortex frontal et d'hippocampe de rat,
- pour les récepteurs sérotoninergiques 5-HT_{1D}, par la mesure du déplacement de la 5-OH-tryptamine, sur des préparations de cortex, striatum et globus pallidus de rat,
- pour les récepteurs sérotoninergiques 5-HT₂, par la mesure du déplacement de l'amino-iodo-kétansérine, sur des préparations de cortex frontal de rat,
- pour les récepteurs sérotoninergiques 5-HT₃, par la mesure du déplacement du BRL 43694, sur des préparations d'aéra postréma de rat.

### RESULTATS :

Certains composés de l'invention présentent une affinité pour les récepteurs β₁, β₂, D₁, D₂, 5-HT_{1D}, 5-HT₂ et 5-HT₃ nettement plus faible que pour les récepteurs 5-HT_{1C}.

### EXEMPLE D : EVALUATION DE L'ACTIVITE ANTAGONISTE DES RECEPTEURS 5-HT_{1A} DES COMPOSES DE L'INVENTION

### PROTOCOLE :

L'évaluation de l'activité antagoniste des récepteurs 5HT_{1A}, des composés de l'invention, a été faite en stimulant, en présence du composé à tester, l'adénylate cyclase par 10 µM de forskoline, en absence ou en présence de 0,1 µM de [8-hydroxy-2-(di-n-propylamino)tétraline] (8-OH-DPAT).
Les produits de l'invention ont été testés pour des gammes de concentrations allant de 10 nM à 10 µM.

### RESULTATS :

Les composés de formule (I) s'opposent de façon compétitive (IC₅₀ < 50 nM) à l'inhibition de l'adénylate cyclase, provoquée par la 8-OH-DPAT (0,1 µM), dans des homogénats d'hippocampe de rat, ce qui traduit une forte activité antagoniste des récepteurs 5-HT_{1A}.

### EXEMPLE E : ETUDE DE L'ACTIVITE ANTIDEPRESSIVE DES COMPOSES DE L'INVENTION

### PRINCIPE :

L'étude des produits est réalisée sur le modèle du "renoncement appris" qui consiste à induire chez l'animal, par une série d'événements aversifs incontrôlables, un déficit lors des tâches d'évitement ultérieures.

### PROTOCOLE :

Ce test a été mis au point par Sherman A.D., Sacquitne J.L., et Petty F. (Pharmacol. Biochem. Behav., 1982,16,449-454). Nous utilisons des rats mâles Wistar d'un poids compris entre 180 et 200 grammes. Les animaux sont maintenus à l'animalerie une semaine avant le test, dans des boîtes en plastique, par groupes de 10, à une température ambiante de 21° C ∓ 1° C, avec libre accès à l'eau et à la nourriture.
Les animaux sont ensuite isolés dans des boîtes de petites dimensions et sont soumis à 60 chocs électriques inévitables (0,8 mA toutes les minutes ∓ 15 secondes). Un groupe de rats témoins ne reçoit pas de chocs électriques.
La capacité des animaux à réaliser un apprentissage d'évitement (passage d'un compartiment à l'autre, afin d'éviter les chocs électriques) est appréciée 48 heures après et pendant 3 jours consécutifs. Lors des séances d'apprentissage, les animaux subissent 2 essais par minute pendant 15 minutes. Le nombre d'échecs d'échappement est noté pour chaque rat. Les animaux sont traités (i.p. : 0,5 cm³/100 g) 6 heures après les chocs inévitables et 4 jours de suite, le matin 30 minutes avant la séance d'apprentissage et le soir entre 18 et 19 h.
Les produits étudiés sont mis en solution dans de l'eau distillée.
Les produits étudiés sont administrés aux doses 0,25 mg/kg/jour.

### RESULTATS :

Le test prouve que certains produits de l'invention diminuent significativement le nombre d'échecs d'échappement ce qui traduit, pour les produits de l'invention, une forte activité de type antidépressive.

### EXEMPLE F : ETUDE DE L'ACTIVITE ANXIOLYTIQUE - TEST DIT DES CAGES CLAIRE/OBSCURE CHEZ LA SOURIS

### PRINCIPE :

Nous nous proposons d'étudier les effets anxiolytiques des composés de l'invention, au moyen du test dit des cages claires/obscures chez la souris.

### PROTOCOLE :

Ce test a été mis au point par Crawley et al. (1981, Pharmacol. Biochem. Behav. 1981, 15 (5), pp 695-9), puis modifié et comportementalement validé.

Il s'agit de deux cages de taille égale (20×20×14 cm) en PVC. L'une est fortement éclairée par une lampe de 100 W(lumière "froide"), l'autre est obscurcie. Les deux cages sont séparées l'une de l'autre au moyen d'un petit tunnel opaque (5×7 cm). Les souris sont individuellement introduites dans la cage éclairée et dès qu'elles ont pénétré pour la première fois dans la cage obscure, on enregistre, au moyen de claviers reliés à un ordinateur, durant 5 minutes, le temps passé par les animaux dans la cage éclairée ainsi que le nombre des transitions entre la cage obscure et la cage éclairée.

Chaque groupe expérimental comprend au minimum 15 animaux.

### RESULTATS :

L'administration, par voie intrapéritonéale des produits de l'invention entraine une augmentation du temps passé par les souris dans la cage éclairée et du nombre des transitions entre la cage obscure et la cage éclairée.

Cette augmentation significative des deux paramètres étudiés montre la remarquable activité anxiolytique des composés de l'invention.

### EXEMPLE G : COMPOSITION PHARMACEUTIQUE

### Comprimés dosés à 5 mg de N-indan-2-yl-8-(2-aminoéthoxy)thiochromane

Formule pour 10 000 comprimés

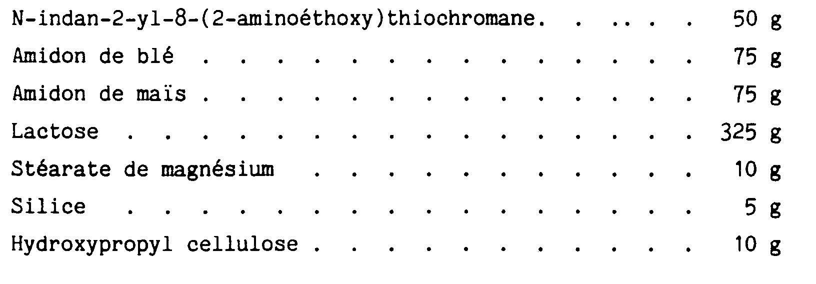

## Revendications

1. Composés de formule (I): 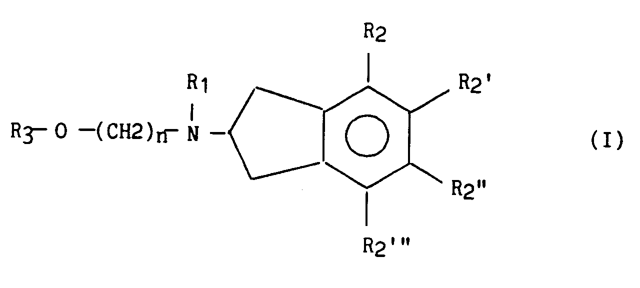 dans laquelle :
- R₁ représente un atome d'hydrogène ou un radical choisi parmi alkyle non substitué ou substitué, cycloalkyle, cycloalkylalkyle, phényl non substitué ou substitué et phénylalkyle non substituté ou substitué
- R₂, R₂', R₂'' et R₂''' représentent indépendamment l'un de l'autre un atome d'hydrogène, ou un radical choisi parmi halogène, alkyle non substitué ou substitué et alkoxy non substitué ou substitué,
- n représente un nombre entier de 1 à 6,
- R₃ représente un groupement de formule (A) : 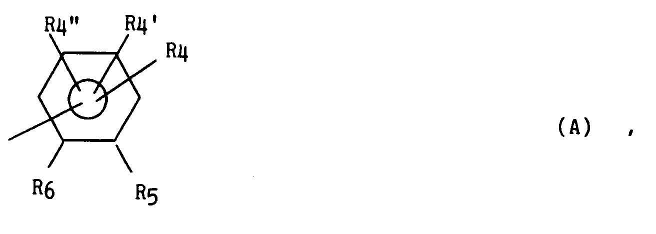 dans laquelle :
- R₄, R₄' et R₄'' représentent indépendemment l'un de l'autre un atome d'hydrogène ou un radical choisi parmi halogène, alkyle non substitué ou substitué et alkoxy inférieur non substitué ou substitué,
- R₅ et R₆ forment ensemble avec le noyau benzène qui les porte un système cyclique E₁ choisi parmi : indène, naphtalène, benzothiophène, benzofurane, indole, benzimidazole, benzopyrane, benzothiopyrane, chromane, thiochromane, quinoléine, isoquinoléine, indazole, 2,3-dihydrol1,4-benzodithiine, quinoxaline, quinazoline, cinnoline, benzothiazole, benzisothiazole, benzoxazole, benzisoxazole, 2,3-dihydrol1,4-benzoxathiine, 1,4-benzoxazine, 1,4-benzothiazine, 1,3-benzodioxole, 1,3-benzodioxane, 1,4-benzodioxane et 1,4-benzodioxine,
étant entendu que la partie du système cyclique E₁ formé par R₅ et R₆ et les 2 atomes de carbone du noyau benzène qui les portent est :
- non hydrogénée ou partiellement hydrogénée,
- et non substituée ou substituée par un ou plusieurs radicaux choisis parmi : halogène, hydroxyle, alkyle inférieur, alkoxy non substitué ou substitué, alkoxycarbonyle inférieur, et carboxyle,
étant entendu que, sauf précisions contraires :
les termes "alkyle" et "alkoxy" signifient des groupements, linéaires ou ramifiés, contenant de 1 à 6 atomes de carbone,
le terme "substitué" associé aux radicaux "alkyle" et "alkoxy" signifie "substitué par un ou plusieurs radicaux choisis parmi halogène, hydroxyle et alkoxy",
le terme "cyclocalkyle" désigne un groupement cyclique de 3 à 8 atomes de carbone,
le terme "substitué" associé aux radicaux "phényle" et "phénylalkyle" signifie que ces radicaux peuvent être substitué sur le noyau phényle par un ou plusieurs substituants choisis parmi halogène, alkyle, alkoxy, hydroxyle et polyhalogénoalkyl,
leurs isomères optiques, ainsi que leurs sels d'addition à un acide ou une base pharmaceutiquement acceptable.

2. Composés de formule (I) selon la revendication 1 dans lesquels R₅ et R₆ forment ensemble avec le noyau benzène qui les porte un système cyclique E₁ choisi parmi naphthalène, benzopyrane, benzothiopyrane, chromane, thiochromane et benzodioxane.

3. Composé selon la revendication 1 qui est le 8-{4-[(N-indan-2yl)amino]butyloxy}thiochromane et ses sels d'addition à un acide pharmaceutiquement acceptable.

4. Composé selon la revendication 1 qui est le 8-{3-[(N-indan-2-yl)amino]propyloxy}thiochromane et ses sels d'addition à un acide pharmaceutiquement acceptable.

5. Composé selon la revendication 1 qui est le 8-{2-[(N-indan-2-yl)amino]éthoxy}thiochromane et ses sels d'addition à un acide pharmaceutiquement acceptable.

6. Composé selon la revendication 1 qui est le 8-{3-[(N-propyl N-indan-2-yl)amino]propyloxy}thiochromane et ses sels d'addition à un acide pharmaceutiquement acceptable.

7. Procédé de préparation des composés de formule (I) selon la revendication 1, caractérisé en ce que l'on fait réagir un dérivé hydroxylé de formule (II) : 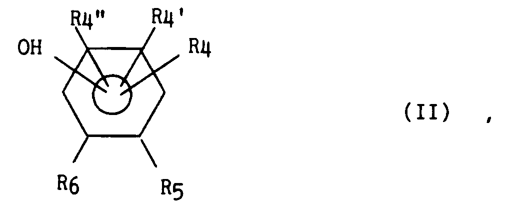 dans laquelle R₄, R₄', R₄'', R₅ et R₆ sont tels que définis dans la revendication 1,
avec un dihalogéncalkyle de formule (III) :
X-(CH₂)ₙ-X', (III)
dans laquelle X et X' sont des atomes d'halogène et n est tel que défini dans la revendication 1, pour donner un composé de formule (IV) : 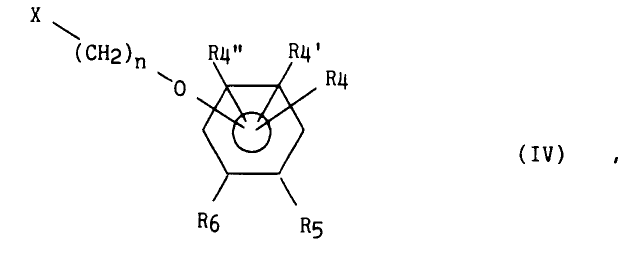 dans laquelle n, X, R₄, R₄', R₄'', R₅, et R₆ sont tels que définis précédemment,
le dit composé de formule (IV) est mis en réaction avec un aminoindane de formule (V) : 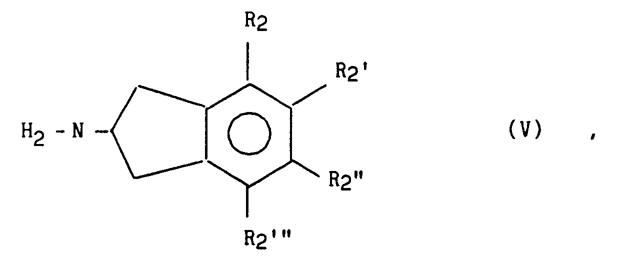 dans laquelle R₂, R₂', R₂'' et R₂''' sont tels que définis dans la revendication 1, pour donner un composé de formule (Ia) : 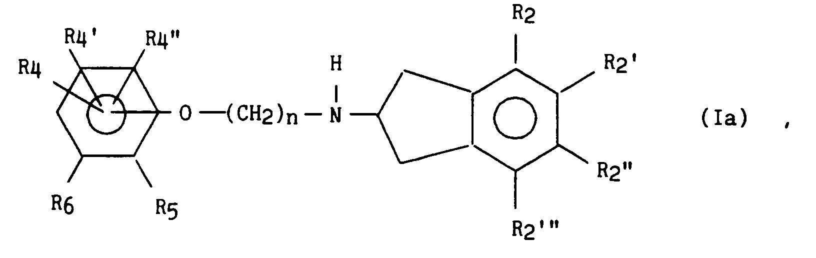 dans laquelle R₂, R₂', R₂'', R₂''', R₄, R₄', R₄'', R₅, R₆ et n sont tels que définis précédemment,
le dit composé de formule (Ia) pouvant être mis en présence d'un halogénoalkyle de formule (VI) :
R₁'-X'', (VI)
dans laquelle X'' est un atome d'halogène et R₁' a la même signification que R₁, tel que défini dans la revendication 1 à l'exception de l'hydrogène, pour donner un composé de formule (Ib) : 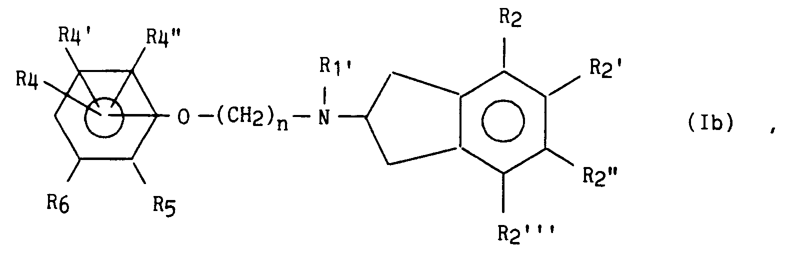 dans laquelle R₁, R₂, R₂', R₂'', R₂''', R₄, R₄', R₄'', R₅, R₆ et n sont tels que définis précédemment,
les composés de formule (Ia) et (Ib) constituent l'ensemble des composés de formule (I) selon la revendication 1 qui peuvent être, si on le désire,
- purifiés suivant une ou plusieurs méthodes de purification choisies parmi la cristallisation, la chromatographie sur colonne de silice, l'extraction, la filtration, et le passage sur charbon et/ou résine,
- séparés, le cas échéant, sous forme pure ou sous forme de mélange, en leurs éventuels isomères optiques,
- et/ou salifiés par un acide ou une base pharmaceutiquement acceptable.

8. Compositions pharmaceutiques contenant un composé de formule (I), selon la revendication 1, ou un de ses sels physiologiquement tolérables, en combinaison avec un ou plusieurs excipients ou véhicules pharmaceutiquement acceptables.

9. Compositions pharmaceutiques selon la revendication 8 utiles dans la prévention et le traitement des troubles liés aux récepteurs 5-HT_{1A} ou 5-HT_{2C}.

10. Compositions selon l'une des revendications 8 ou 9 utiles dans le traitement et la prévention du stress, de l'anxiété, de la dépression, des psychoses, de la schizophrénie, de la douleur, des troubles du comportement alimentaire et sexuel et des troubles du sommeil.

## Claims

1. Compounds of formula (I): 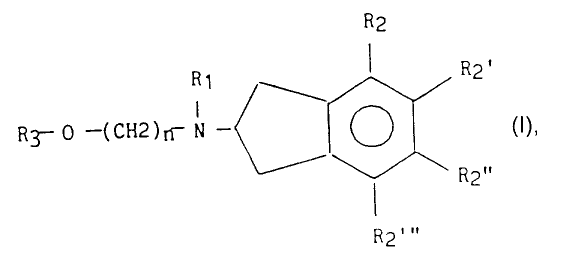 wherein:
- R₁ represents a hydrogen atom or a radical selected from unsubstituted or substituted alkyl, cycloalkyl, cycloalkylalkyl, unsubstituted or substituted phenyl and unsubstituted or substituted phenylalkyl,
- R₂, R₂', R₂'' and R₂''', each independently of the others, represents a hydrogen atom or a radical selected from halogen, unsubstituted or substituted alkyl and unsubstituted or substituted alkoxy,
- n represents an integer from 1 to 6,
- R₃ represents a group of formula (A): 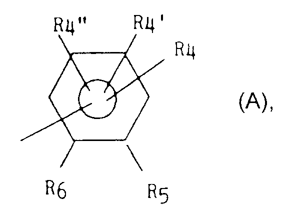 wherein:
- R₄, R₄' and R₄'', each independently of the others, represents a hydrogen atom or a radical selected from halogen, unsubstituted or substituted alkyl and unsubstituted or substituted lower alkoxy,
- R₅ and R₆, together with the benzene nucleus carrying them, form a ring system E₁ selected from: indene, naphthalene, benzothiophene, benzofuran, indole, benzimidazole, benzopyran, benzothiopyran, chroman, thiochroman, quinoline, isoquinoline, indazole, 2,3-dihydro-1,4-benzodithiin, quinoxaline, quinazoline, cinnoline, benzothiazole, benzisothiazole, benzoxazole, benzisoxazole, 2,3-dihydro-1,4-benzoxathiin, 1,4-benzoxazine, 1,4-benzothiazine, 1,3-benzodioxole, 1,3-benzodioxane, 1,4-benzodioxane and 1,4-benzodioxin,
it being understood that the portion of the ring system E₁ formed by R₅ and R₆ and the 2 carbon atoms of the benzene nucleus carrying them:
- is not hydrogenated or is partially hydrogenated,
- and is unsubstituted or is substituted by one or more radicals selected from: halogen, hydroxy, lower alkyl, unsubstituted or substituted alkoxy, lower alkoxycarbonyl and carboxy,
it being understood, unless specified otherwise, that:
the terms "alkyl" and "alkoxy" indicate linear or branched groups having from 1 to 6 carbon atoms,
the term "substituted" associated with "alkyl' and "alkoxy" radicals indicates "substituted by one or more radicals selected from halogen, hydroxy and alkoxy",
the term "cycloalkyl" denotes a cyclic group having from 3 to 8 carbon atoms,
the term "substituted" associated with "phenyl" and "phenylalkyl" indicates that those radicals may be substituted on the phenyl nucleus by one or more substituents selected from halogen, alkyl, alkoxy, hydroxy and polyhaloalkyl,
their optical isomers, and addition salts thereof with a pharmaceutically acceptable acid or base.

2. Compounds of formula (I) according to claim 1, wherein R₅ and R₆, together with the benzene nucleus carrying them, form a ring system E₁ selected from naphthalene, benzopyran, benzothiopyran, chroman, thiochroman and benzodioxane.

3. Compound according to claim 1 that is 8-{4-[(N-indan-2-yl)amino]butoxy}thiochroman and addition salts thereof with a pharmaceutically acceptable acid.

4. Compound according to claim 1 that is 8-{3-[(N-indan-2-yl)amino]propoxy}thiochroman and addition salts thereof with a pharmaceutically acceptable acid.

5. Compound according to claim 1 that is 8-{2-[(N-indan-2-yl)amino]ethoxy}thiochroman and addition salts thereof with a pharmaceutically acceptable acid.

6. Compound according to claim 1 that is 8-{3-[(N-propyl-N-indan-2-yl)amino]propoxy}-thiochroman and addition salts thereof with a pharmaceutically acceptable acid.

7. Process for the preparation of compounds of formula (I) according to claim 1, characterised in that a hydroxylated compound of formula (II): 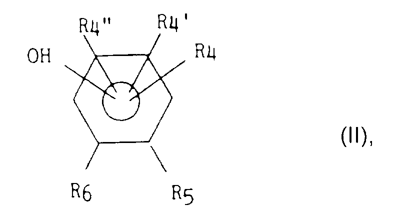 wherein R₄, R₄', R₄'', R₅ and R₆ are as defined in claim 1,
is reacted with a dihaloalkyl of formula (III):
X― (CH₂)ₙ―X', (III)
wherein X and X' are halogen atoms and n is as defined in claim 1, to yield a compound of formula (IV): 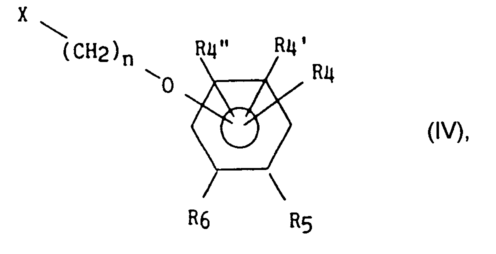 wherein n, X, R₄, R₄', R₄'', R₅ and R₆ are as defined previously,
which compound of formula (IV) is reacted with an aminoindane of formula (V): 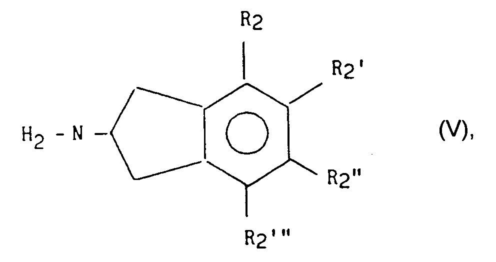 wherein R₂, R₂', R₂'' and R₂''' are as defined in claim 1, to yield a compound of formula (Ia): 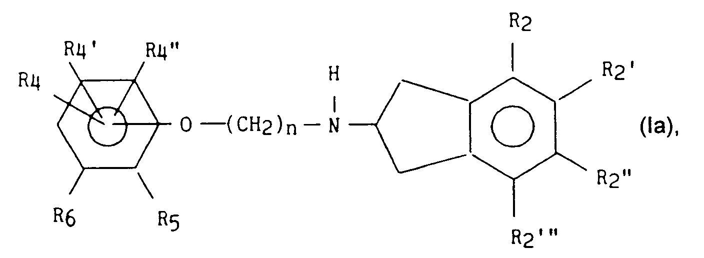 wherein R₂, R₂', R₂'', R₂''', R₄, R₄', R₄'', R₅, R₆ and n are as defined previously,
the said compound of formula (Ia) optionally being placed in the presence of a haloalkyl of formula (VI):
R₁'―X'', (VI)
wherein X'' is a halogen atom and R₁' has the same meaning as R₁ as defined in claim 1 with the exception of hydrogen, to yield a compound of formula (Ib): 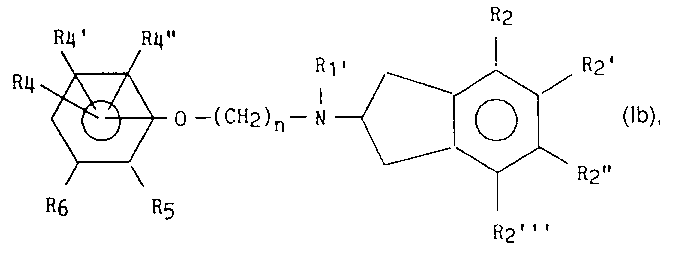 wherein R₁', R₂, R₂', R₂'', R₂''', R₄, R₄', R₄'', R₅, R₆ and n are as defined previously,
which compounds of formula (Ia) and (Ib) constitute the totality of the compounds of formula (I) according to claim 1 and may, if desired, be
- purified by one or more purification methods selected from crystallisation, chromatography on a silica column, extraction, filtration and passage over charcoal and/or resin,
- separated, where applicable, in pure form or in the form of a mixture, into their possible optical isomers,
- and/or converted into salts with a pharmaceutically acceptable acid or base.

8. Pharmaceutical compositions comprising a compound of formula (I) according to claim 1, or a physiologically tolerable salt thereof, in combination with one or more pharmaceutically acceptable excipients or carriers.

9. Pharmaceutical compositions according to claim 8 for use in the prevention and treatment of disorders associated with 5-HT_{1A} or 5-HT_{2C} receptors.

10. Compositions according to either claim 8 or claim 9 for use in the treatment and prevention of stress, anxiety, depression, psychoses, schizophrenia, pain, eating disorders, disorders of sexual behaviour and sleep disorders.

## Patentansprüche

1. Verbindungen der Formel (I): 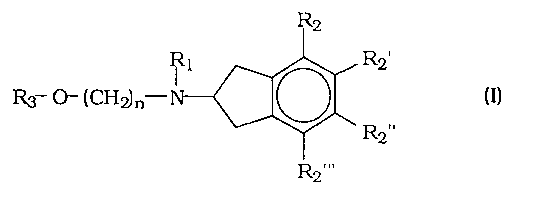 in der:
- R₁ ein Wasserstoffatom oder eine Gruppe ausgewählt aus nichtsubstituiertem oder substituiertem Alkyl, Cycloalkyl, Cycloalkylalkyl, nichtsubstituiertem oder substituiertem Phenyl und nichtsubstituiertem oder substituiertem Phenylalkyl,
- R₂, R₂'' und R₂''' unabhängig voneinander ein Wasserstoffatom oder eine Gruppe ausgewählt aus Halogen, unsubstituiertem oder substituiertem Alkyl und unsubstituiertem oder substituiertem Alkoxy,
- n eine ganze Zahl mit einem Wert von 1 bis 6,
- R₃ eine Gruppe der Formel (A): 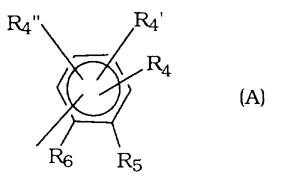 in der
- R₄, R₄' und R₄'' unabhängig voneinander ein Wasserstoffatom oder eine Gruppe ausgewählt aus Halogen, unsubstituiertem oder substituiertem Alkyl und unsubstituiertem oder substituiertem Alkoxy und
- R₅ und R₆ gemeinsam mit dem sie tragenden Benzolkern ein cyclisches System E₁ darstellen, welches aus: Inden, Naphthalin, Benzothiophen, Benzofuran, Indol, Benzimidazol, Benzopyran, Benzothiopyran, Chroman, Thiochroman, Chinolin, Isochinolin, Indazol, 2,3-Dihydro-1,4-benzodithiin, Chinoxalin, Chinazolin, Cinnolin, Benzothiazol, Benzisothiazol, Benzoxazol, Benzisoxazol, 2,3-Dihydro-1,4-benzoxathiin, 1,4-Benzoxazin, 1,4-Benzothiazin, 1,3-Benzodioxol, 1,3-Benzodioxan, 1,4-Benzodioxan und 1,4-Benzodioxin ausgewählt ist,
mit der Maßgabe bedeuten, daß der Teil des durch R₅ und R₆ und die beiden sie tragenden Kohlenstoffatome des Benzolkerns gebildeten cyclischen Systems E₁:
- nicht hydriert oder teilweise hydriert,
- und nichtsubstituiert oder durch eine oder mehrere Gruppen ausgewählt aus: Halogen, Hydroxyl, Niedrigalkyl, unsubstituiertem oder substituiertem Alkoxy, Niedrigalkoxycarbonyl und Carboxyl substituiert ist,
wobei, wenn nichts anderes angegeben ist:
die Begriffe "Alkyl" und "Alkoxy" für geradkettige oder verzweigte Gruppen, die 1 bis 6 Kohlenstoffatome enthalten, stehen,
der Begriff "substituiert" unter Bezugnahme auf die Gruppen "Alkyl" und "Alkoxy" für "substituiert durch eine oder mehrere Gruppen ausgewählt aus Halogen, Hydroxyl und Alkoxy" steht,
der Begriff "Cycloalkyl" für eine cyclische Gruppe mit 3 bis 8 Kohlenstoffatomen steht,
der Begriff "substituiert" unter Bezugnahme auf die Gruppen "Phenyl" und "Phenylalkyl" bedeutet, daß diese Gruppen am Phenylkern durch einen oder mehrere Substituenten ausgewählt aus Halogen, Alkyl. Alkoxy, Hydroxyl und Polyhalogenalkyl substituiert sein können,
deren optische Isomeren sowie deren Additionssalze mit einer pharmazeutisch annehmbaren Säure oder Base.

2. Verbindungen der Formel (I) nach Anspruch 1, worin R₅ und R₆ gemeinsam mit dem sie tragenden Benzolkern ein cyclisches System E₁ bilden, welches aus Naphthalin, Benzopyran, Benzothiopyran, Chroman, Thiochroman und Benzodioxan ausgewählt ist.

3. Verbindung nach Anspruch 1, nämlich 8-{4-[(N-Indan-2-yl)-amino]-butyloxy}-thiochroman und dessen Additionssalze mit einer pharmazeutisch annehmbaren Säure.

4. Verbindung nach Anspruch 1, nämlich 8-{3-[(N-Indan-2-yl)-amino]-propyloxy}-thiochroman und dessen Additionssalze mit einer pharmazeutisch annehmbaren Säure.

5. Verbindung nach Anspruch 1, nämlich 8-{2-[(N-Indan-2-yl)-amino]-ethoxy}-thiochroman und dessen Additionssalze mit einer pharmazeutisch annehmbaren Säure.

6. Verbindung nach Anspruch 1, nämlich 8-{3-[(N-Propyl-N-indan-2-yl)-amino]-propyloxy}-thiochroman und dessen Additionssalze mit einer pharmazeutisch annehmbaren Säure.

7. Verfahren zur Herstellung der Verbindungen der Formel (I) nach Anspruch 1, dadurch gekennzeichnet, daß man ein hydroxyliertes Derivat der Formel (II): 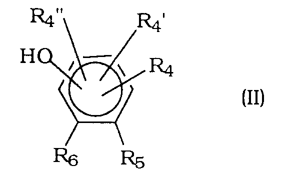 In der R₄, R₄', R₄'', R₅ und R₆ die in Anspruch 1 angegebenen Bedeutungen besitzen,
mit einem Dihalogenalkyl der Formel (III):
X―(CH₂)ₙ―X, (III)
in der X und X' Halogenatome bedeuten und n die in Anspruch 1 angegebenen Bedeutungen besitzt, umsetzt zur Bildung einer Verbindung der Formel (IV): 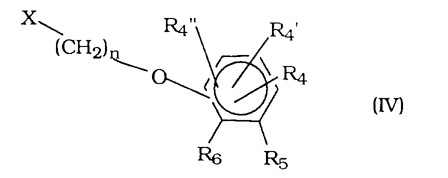 in der n, X, R₄, R₄', R₄'', R₅ und R₆ die oben angegebenen Bedeutungen besitzen, welche Verbindung der Formel (IV) mit einem Aminoindan der Formel (V): 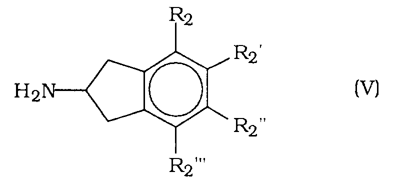 in der R₂, R₂', R₂'' und R₂''' die in Anspruch 1 angegebenen Bedeutungen besitzen, umsetzt zur Bildung einer Verbindung der Formel (Ia): 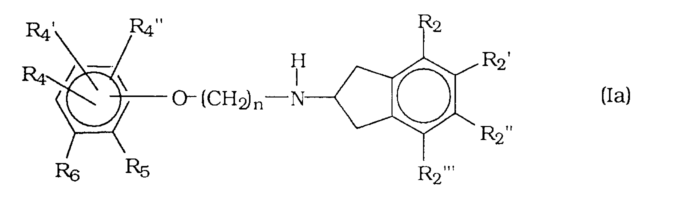 in der R₂, R₂', R₂'', R2''', R₄, R₄', R₄'', R₅, R₆ und n die oben angegebenen Bedeutungen besitzen,
welche Verbindung der Formel (Ia) mit einem Halogenalkyl der Formel (VI):
R₁' - X'' (VI)
in der X'' ein Halogenatom darstellt und R₁' die Bedeutungen von R₁ besitzt, wie sie in Anspruch 1 definiert sind mit Ausnahme von Wasserstoff, in Kontakt gebracht wird zur Bildung einer Verbindung der Formel (Ib): 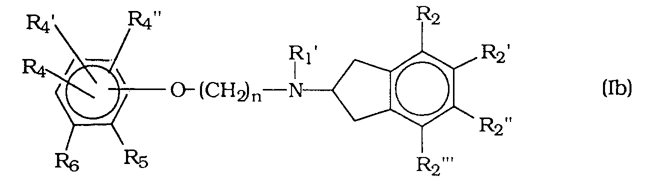 in der R₁, R₂, R₂', R₂'', R₂''', R₄, R₄', R₄'', R₅, R₆ und n die oben angegebenen Bedeutungen besitzen,
welche Verbindungen der Formeln (Ia) und (Ib) gemeinsam die Verbindungen der Formel (I) nach Anspruch 1 bilden, welche gewünschtenfalls
- mit Hilfe einer oder mehrerer Reinigungsmethoden ausgewählt aus Kristallisation, Säulenchromatographie über Siliciumdioxid, Extraktion, Filtration und Überführung über Aktivkohle und/oder ein Harz gereinigt werden können,
- gegebenenfalls in ihre eventuellen optischen Isomeren in reiner Form oder in Form einer Mischung getrennt werden können
- und/oder mit einer pharmazeutisch annehmbaren Säure oder Base in ein Salz überführt werden können.

8. Pharmazeutische Zubereitungen enthaltend eine Verbindung der Formel (I) nach Anspruch 1 oder eines ihrer physiologisch verträglichen Salze in Kombination mit einem oder mehreren pharmazeutisch annehmbaren Trägermaterialien oder Bindemitteln.

9. Pharmazeutische Zubereitungen nach Anspruch 8 zur Vorbeugung und zur Behandlung von Störungen, die mit den Rezeptoren 5-HT_{1A} oder 5-HT_{2C} verknüpft sind.

10. Zubereitungen nach einem der Ansprüche 8 oder 9 zur Behandlung oder zur Vorbeugung von Streß, der Angst, der Depression, von Psychosen, der Schizophrenie, von Schmerzen, von Ernährungs- und Sexualverhaltensstörungen und von Schlafstörungen.
